(19) Europäisches Patentamt
European Patent Office
Office européen des brevets

(11) Veröffentlichungsnummer: **0 353 452 A1**

(12) **EUROPÄISCHE PATENTANMELDUNG**

(21) Anmeldenummer: **89111560.2**

(51) Int. Cl.4: **A61K 7/13**

(22) Anmeldetag: **24.06.89**

Die Bezeichnung der Erfindung wurde geändert (Richtlinien für die Prüfung im EPA, A-III, 7.3).

(30) Priorität: **02.07.88 DE 3822449**

(43) Veröffentlichungstag der Anmeldung: **07.02.90 Patentblatt 90/06**

(84) Benannte Vertragsstaaten: **ES GR**

(71) Anmelder: **Henkel Kommanditgesellschaft auf Aktien**
**Postfach 1100 Henkelstrasse 67**
**D-4000 Düsseldorf 1(DE)**

(72) Erfinder: **Konrad, Günther, Dr.**
**Feuerbachweg 12**
**D-4010 Hilden(DE)**
Erfinder: **Lieske, Edgar**
**Hunsrückenstrasse 40**
**D-4000 Düsseldorf(DE)**
Erfinder: **Möller, Hinrich, Dr.**
**Schumannstrasse 11**
**D-4019 Monheim(DE)**

(54) **Oxidationshaarfärbemittel.**

(57) Haarfärbemittel, enthaltend Oxidationsfarbstoffvorprodukte mit üblichen Entwicklerkomponenten in einem kosmetischen Träger, die als Kupplerkomponenten Dihydroxybenzamide der allgemeinen Formel I

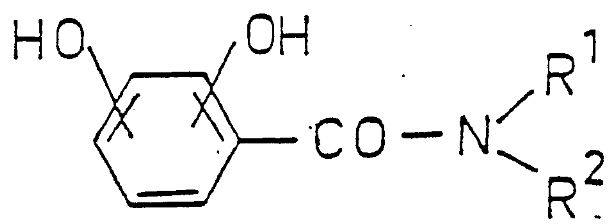

(I)

enthalten, in der $R^1$ und $R^2$ unabhängig voneinander Wasserstoff oder Alkylgruppen mit 1-4 Kohlenstoffatomen, Hydroxyalkyl-oder Aminoalkyl-Guppen, jeweils mit 2-4 Kohlenstoffatomen, Pyridyl-Gruppen oder Gruppen der Formel (II)

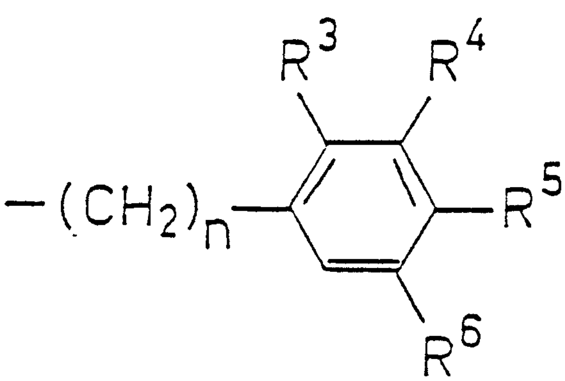

(II)

EP 0 353 452 A1

darstellen, wobei n = 0 - 4 ist und $R^3$-$R^6$ für Wasserstoff oder Hydroxylgruppen, Alkyl- oder Alkoxygruppen mit 1-4 Kohlenstoffatomen, Hydroxyalkyl- oder Aminoalkyl-Gruppen, jeweils mit 2-4 Kohlenstoffatomen, für $NR^7R^8$-Gruppen, mit $R^7$ und $R^8$ für Wasserstoff, oder Alkylgruppen mit 1-4 Kohlenstoffatomen, Hydroxyalkyl-oder Aminoalkylgruppen, jeweils mit 2-4 Kohlenstoffatomen, oder für die Carboxylgruppen stehen, wobei wenigstens zwei der Reste $R^3$-$R^6$ Wasserstoffatome sind und nicht mehr als ein Rest eine Carboxylgruppe ist, zeichnen sich durch eine hohe Licht- und Kaltwell-Echtheit der gebildeten Haarfarbstoffe und deren verbesserte Egalisierung, d. h. deren sehr gleichmäßige Verteilung auf dem Haar vom Haaransatz bis zur Haarspitze, aus.

## Oxidationshaarfärbemittel mit neuen Kupplern

Gegenstand der Erfindung sind Haarfärbemittel auf der Basis von Oxidationsfarbstoffen. Solche Haarfärbemittel enthalten Oxidationsfarbstoffvorprodukte in einem kosmetischen Träger. Als Oxidationsfarbstoffvorprodukte werden Entwicklersubstanzen und Kupplersubstanzen eingesetzt, die unter dem Einfluß von Oxidationsmitteln oder von Luftsauerstoff Farbstoffe ausbilden. Als kosmetische Träger für die Oxidationsfarbstoffvorprodukte dienen Cremes, Emulsionen, Gele, Shampoos, Schaumaerosole oder andere Zubereitungen, die für die Anwendung auf dem Haar geeignet sind.

Für das Färben von Haaren spielen die sogenannten Oxidationsfarben, die durch oxidative Kupplung einer oder mehrerer Entwicklerkomponenten untereinander oder mit einer oder mehreren Kupplerkomponenten entstehen, wegen ihrer intensiven Farben und guten Echtheitseigenschaften eine bevorzugte Rolle. Als Entwicklersubstanzen werden üblicherweise primäre aromatische Amine mit einer weiteren in Para- oder Orthoposition befindlichen freien oder substituierten Hydroxy- oder Aminogruppe, ferner Diaminopyridinderivate, heterocyclische Hydrazonderivate, 4-Aminopyrazolonderivate und Tetraaminopyrimidine eingesetzt. Als Kupplersubstanzen werden beispielsweise m-Phenylendiaminderivate, Naphthole, Resorcinderivate und Pyrazolone verwendet.

Gute Oxidationshaarfarbstoffvorprodukte müssen in erster Linie folgende Voraussetzungen erfüllen: Sie müssen bei der oxidativen Kupplung die gewünschten Farbnuancen in ausreichender Intensität ausbilden. Sie müssen ferner ein gutes Aufziehvermögen auf menschlichem Haar besitzen, ohne die Kopfhaut zu stark anzufärben. Die damit erzeugten Färbungen müssen eine hohe Stabilität gegen Abrieb, Wärme, Licht und die bei der Dauerwellung des Haars verwendeten Chemikalien aufweisen. Schließlich sollen die Oxidationshaarfarbstoffvorprodukte in toxikologischer und dermatologischer Hinsicht unbedenklich sein.

Es wurde nun gefunden, daß Haarfärbemittel, enthaltend Oxidationsfarbstoffvorprodukte mit üblichen Entwicklerkomponenten in einem kosmetischen Träger, die als Kupplerkomponenten Dihydroxybenzamide der allgemeinen Formel (I)

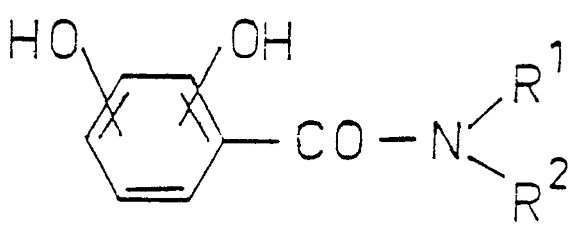

(I)

enthalten, in der $R^1$ und $R^2$ unabhängig voneinander Wasserstoff oder Alkylgruppen mit 1-4 Kohlenstoffatomen, Hydroxyalkyl- oder Aminoalkyl-Gruppen, jeweils mit 2-4 Kohlenstoffatomen, Pyridyl-Gruppen, oder Gruppen der Formel (II)

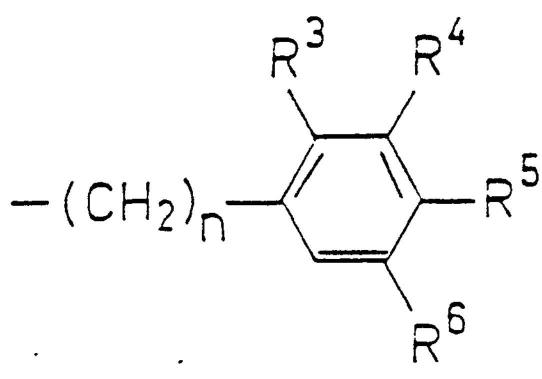

(II)

darstellen, wobei n = 0 - 4 ist und $R^3$-$R^6$ für Wasserstoff oder Hydroxylgruppen, Alkyl- oder Alkoxygruppen mit 1-4 Kohlenstoffatomen, Hydroxyalkyl- oder Aminoalkyl-Gruppen, jeweils mit 2-4 Kohlenstoffatomen, für $NR^7R^8$-Gruppen, mit $R^7$ und $R^8$ für Wasserstoff, oder Alkyl mit 1-4 Kohlenstoffatomen, Hydroxyalkyl oder

Aminoalkyl, jeweils mit 2-4 Kohlenstoffatomen, oder für die Carboxylgruppe stehen, wobei wenigstens zwei der Reste $R^3$-$R^6$ Wasserstoffatome sind und nicht mehr als ein Rest eine Carboxylgruppe ist, diesen Anforderungen im hohem Maße genügen. Insbesondere zeichnen sich die gebildeten Haarfarbstoffe durch eine hohe Licht- und Kaltwell-Echtheit und durch eine verbesserte Egalisierung, d. h. eine sehr gleichmäßige Verteilung auf dem Haar von Haaransatz bis Haarspitze, aus.

Die erfindungsgemäßen Kupplerkomponenten sind bereits in der Literatur, beispielsweise in der deutschen Offenlegungsschrift 27 10 653, der deutschen Auslegeschrift 10 64 074, der europäischen Patentanmeldung 161 655, der britischen Patentschrift 872 683, der französischen Patentschrift 1 571 198 und der belgischen Patentschrift 813 815 beschrieben. Sie sind prinzipiell durch Umsetzung von Dihydroxybenzoesäure, in Form des Säurechlorides, bei dem die Hydroxygruppen acetyliert sind, mit den entsprechenden Aminen herstellbar. Eine entsprechende Arbeitsvorschrift findet sich beispeilsweise in der deutschen Auslegeschrift 10 64 074.

Von den durch Formel (I) beschriebenen Dihydroxybenzamiden sind die 2,4-Dihydroxybenzamide bevorzugt.

Es ist weiterhin bevorzugt, solche Dihydroxybenzamide der allgemeinen Struktur (I) zu verwenden, bei denen $R^1$ für ein Wasserstoffatom steht. Im Rahmen dieser genannten Substanzen ist es wiederum bevorzugt, solche Verbindungen einzusetzen, bei denen $R^2$ steht für Wasserstoff, eine Methylgruppe, eine Pyridyl-Gruppe, oder eine Gruppe der Formel (II), wobei $R^3$-$R^6$ stehen für Wasserstoff, Methoxygruppen, Aminogruppen, N,N-Dimethylaminogruppen oder die Carboxylgruppe, wobei wenigstens zwei der Gruppen $R^3$-$R^6$ Wasserstoffatome sind und nicht mehr als eine Gruppe eine Carboxylgruppe ist.

Die Dihydroxybenzamide werden in den erfindungsgemäßen Haarfärbemitteln bevorzugt in freier Form eingesetzt; es können aber auch, wenn saure oder basische Gruppen enthalten sind, die entsprechenden Salze eingesetzt werden. Die Verbindungen mit einer -COOH-Gruppe können etwa in Form der Alkali- oder Ammoniumsalze, Verbindungen mit Amin-Gruppen beispielsweise auch in Form der Hydrochloride eingesetzt werden.

Besonders geeignete Kupplungskomponenten sind N-Phenyl-2,4-dihydroxybenzamid, N-(2′-Methoxyphenyl)-2,4-dihydroxybenzamid, N-(3′-Methoxyphenyl)-2,4-dihydroxybenzamid, N-(4′-Methoxyphenyl)-2,4-dihydroxybenzamid, N-(4′-Carboxyphenyl)-2,4-dihydroxybenzamid, N-(2′-Pyridyl)-2,4-dihydroxybenzamid, N-(3′-Pyridyl)-2,4-dihydroxybenzamid, N-(2′,5′-Dimethoxyphenyl)-2,4-dihydroxybenzamid, N-(3′,5′-Dimethoxyphenyl)-2,4-dihydroxybenzamid, N-(2′-Methoxy-5′-amino-phenyl)-2,4-dihydroxybenzamid, N-(4′-N,N,-Dimethylamino)phenyl)-2,4-dihydroxybenzamid, N-(4′-Hydroxyphenyl)-2,4-dihydroxybenzamid, N-Methyl-2,4-dihydroxybenzamid, N-Benzyl-2,4-dihydroxybenzamid sowie das unsubstituierte 2,4-Dihydroxybenzamid.

Die erfindungsgemäßen Haarfärbemittel können neben den Dihydroxybenzamiden der allgemeinen Formel (I) auch andere bekannte Kupplersubstanzen enthalten, die zur Modifizierung der Farbnuancen und zur Erzeugung natürlicher Farbtöne erforderlich sind. Solche üblichen Kupplerverbindungen sind z.B. andere m-Phenylendiamine, z.B. 2,4-Diaminophenyl-2-hydroxyethylether, oder N-(2,4-Dihydroxybenzyliden)-Aminoverbindungen, Phenole, Resorcine, m-Aminophenole, Naphthole oder Pyrazolone.

Gegebenenfalls können auch direktziehende Farbstoffe zusätzlich zur weiteren Modifizierung der Farbnuancen eingesetzt werden. Solche direktziehenden Farbstoffe sind z.B. Nitrophenylendiamine, Nitroaminophenole, Anthrachinonfarbstoffe oder Indophenole.

Als Entwicklersubstanzen können in den erfindungsgemäßen Haarfärbemitteln z.B. aromatische Amine mit einer oder mehreren weiteren $NH_2$-Gruppen, NHR-Gruppen oder $NR_2$-Gruppen, wobei R eine Alkylgruppe mit 1 - 4 C-Atomen oder eine Hydroxyalkylgruppe oder eine Aminoalkylgruppe mit 2 - 4 C-Atomen darstellt, Aminophenole, Aminophenolether und/oder Diaminopyridinderivate verwendet werden. Solche Entwicklersubstanzen sind z.B. p-Phenylendiamin, p-Toluylendiamin, N-Methyl-p-phenylendiamin, N,N-Dimethyl-p-phenylendiamin, N-Hydroxyethyl-p-phenylendiamin, N,N-Bis(2-hydroxyethyl)p-phenylendiamin, N-Ethyl-N-(2-hydroxyethyl)p-phenylendiamin, N,N-Diethyl-2-methyl-p-phenylendiamin, 2-Chlor-p-phenylendiamin, 2,6-Dichlor-p-phenylendiamin, 2-Chlor-6-methyl-p-phenylendiamin, 2-Methoxy-p-phenylendiamin, 2,5-Diaminoanisol, 6-Methoxy-3-methyl-p-phenylendiamin, N-(2-Methoxyethyl)-p-phenylendiamin, N-(2-Hydroxypropyl)-p-phenylendiamin, N-Butyl-N-sulfobutyl-p-phenylen diamin, N-(p-Aminophenyl)-N′,N′-bis-(β-hydroxyethyl)-1.3-diaminopropan oder deren Salze mit anorganischen oder organischen Säuren.

Besonders geeignete Entwicklersubstanzen für die erfindungsgemäßen Kupplerkomponenten sind 2,4,5,6-Tetraaminopyrimidine der allgemeinen Formel (III),

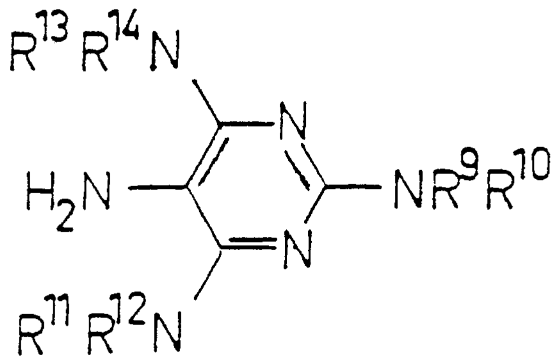

(III)

in der $R^9$-$R^{14}$ Wasserstoff, einen Alkylrest mit 1-4 Kohlenstoffatomen, den Rest $-(CH_2)_m$-X, in dem m = 1-4 und X eine Hydroxylgruppe, ein Halogenatom, eine $-NR^{15}R^{16}$-Gruppe, wobei $R^{15}$ und $R^{16}$ Wasserstoff oder Alkylreste mit 1-4 Kohlenstoffatomen bedeuten können, oder einen gegebenenfalls substituierten Arylrest darstellt, oder unter der Bedingung, daß $R^{11}$-$R^{14}$ Wasserstoffatome sind, $-NR^9R^{10}$ steht für eine Gruppe (IV),

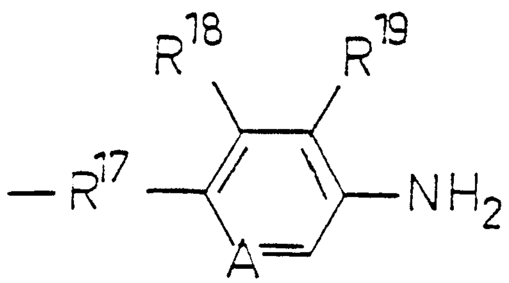

(IV)

in der $R^{17}$ eine Gruppe $-NH-(CH_2)_p-NH-$, worin p = 2 - 4 ist, eine Gruppe $-NH-CH_2-CH(OH)-CH_2-NH-$ oder eine Gruppe

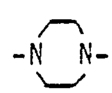

ist, $R^{18}$ und $R^{19}$ unabhängig voneinander Wasserstoff, Chlor oder eine Gruppe $-OR^{20}$ sind, worin $R^{20}$ eine Alkylgruppe mit 1 bis 4 C-Atomen ist, und A eine CH-Gruppe oder ein Stickstoffatom ist, oder $R^9$ und $R^{10}$ und/oder $R^{11}$ und $R^{12}$ und/oder $R^{13}$ und $R^{14}$ mit dem jeweiligen Stickstoffatom einen heterocyclischen, 5- oder 6-gliedrigen Ring mit einem oder zwei Stickstoffatomen oder einem Stickstoff- und einem Sauerstoffatom bilden können, sowie deren Salze mit anorganischen oder organischen Säuren.

Geeignete Substanzen aus dieser Verbindungsklasse sind z.B. 4,5-Diamino-2,6-bis-methylaminopyrimidin, 2,5-Diamino-4-diethylamino-6-methylaminopyrimidin, 2,4,5-Triamino-6-anilino-pyrimidin, 2,4,5-Triamino-6-morpholino-pyrimidin und 2,4,5-Triamino-6-(2-hydroxyethyl)-aminopyrimidin.

Ganz besonders geeignet als Entwicklerkomponente ist das unsubstituierte 2,4,5,6-Tetraaminopyrimidin.

Es ist nicht erforderlich, daß die erfindungsgemäßen Verbindungen der Formel (I) sowie die sonst in den Haarfärbemitteln vorhandenen Oxidationsfarbstoffvorprodukte oder direkt ziehenden Farbstoffe einheitliche chemische Verbindungen darstellen. Vielmehr können diese auch Gemische der erfindungsgemäß einzusetzenden Kuppler-oder Entwicklersubstanzen sein.

Zu den erfindungsgemäßen Haarfärbemitteln werden Verbindungen der Formel (I) und die gegebenenfalls zusätzlich vorhandenen bekannten Kupplersubstanzen im allgemeinen in etwa molaren Mengen, bezogen auf die verwendeten Entwicklersubstanzen, eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklersubstanzen und Kupplersubstanzen in einem Molverhältnis von 1 : 0,5 bis 1 : 2 enthalten sein können.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt am Haar gewünscht wird. Als Oxidationsmittel kommen insbesondere Wasserstoffperoxid oder dessen Anlagerungsprodukte an Harnstoff, Melamin oder Natriumborat sowie Gemische aus derartigen Wasserstoffperoxidanlagerungsprodukten mit Kaliumperoxiddisulfat in Betracht.

Zur Herstellung der erfindungsgemäßen Haarfärbemittel werden die Oxidationsfarbstoffvorprodukte in einen geeigneten kosmetischen Träger eingearbeitet. Solche Träger sind z.B. Cremes, Emulsionen, Gele oder auch tensidhaltige, schäumende Lösungen, z.B. Shampoos oder andere Zubereitungen, die für die Anwendung auf dem Haar geeignet sind. Übliche Bestandteile solcher kosmetischer Zubereitungen sind z.B. Netz- und Emulgiermittel wie anionische, nichtionische oder ampholytische Tenside, z.B. Fettalkoholsulfate, Alkansulfonate, alpha-Olefinsulfonate, Fettalkoholpolyglykolethersulfate, Ethylenoxidanlagerungsprodukte an Fettalkohole, Fettsäuren und Alkylphenole, Sorbitanfettsäureester und Fettsäurepartialglyceride, Fettsäurealkanolamide sowie Verdickungsmittel wie z.B. Methyl- oder Hydroxyethylcellulose, Stärke, Fettalkohole, Paraffinöle, Fettsäuren, ferner Parfümöle und haarpflegende Zusätze, wie z.B. wasserlösliche kationische Polymere, Proteinderivate, Pantothensäure und Cholesterin.

Die Bestandteile der kosmetischen Träger werden zur Herstellung der erfindungsgemäßen Haarfärbemittel in für diese Zwecke üblichen Mengen eingesetzt, z.B. werden Emulgiermittel in Konzentrationen von 0,5 - 30 Gew.-% und Verdickungsmittel in Konzentrationen von 0,1 - 25 Gew.-% des gesamten Färbemittels eingesetzt. Die Oxidationsfarbstoffvorprodukte werden in Mengen von 0,2 - 5 Gew.-%, vorzugsweise 1 - 3 Gew.-% des gesamten Färbemittels in den Träger eingemischt. Der Gehalt an Dihydroxybenzamiden der Formel (I) kan in den erfindungsgemäßen Haarfärbemitteln etwa 0,05 - 10 Millimol pro 100 g des Haarfärbemittels betragen.

Die Anwendung der erfindungsgemäßen Haarfärbemittel kann, unabhängig von der Art der kosmetischen Zubereitung, z.B. als Creme, Gel oder Shampoo, im schwach sauren, neutralen oder alkalischen Milieu erfolgen. Bevorzugt ist die Anwendung der Haarfärbemittel in einem pH-Bereich von 6 - 10. Die Anwendungstemperaturen können in einem Bereich zwischen 15° C und 40° C liegen. Nach einer Einwirkungszeit von ca. 30 Minuten wird das Haarfärbemittel durch Ausspülen von dem zu färbenden Haar entfernt. Danach wird das Haar mit einem milden Shampoo nachgewaschen und getrocknet. Das Nachwaschen mit einem Shampoo entfällt, wenn ein stark tensidhaltiger Träger, z.B. ein Färbeshampoo, verwendet wurde.

Die nachfolgenden Beispiele sollen die Erfindungsgegenstand näher erläutern ohne ihn jedoch hierauf zu beschränken.

## Beispiele

### I. Herstellung von Dihydroxybenzamiden der Formel I

Aus Diacetoxybenzoesäure wurde durch Reaktion mit Phosphorpentachlorid Diacetoxybenzoylchlorid hergestellt, das anschließend mit dem entsprechenden Amin umgesetzt wurde. Durch Verseifen mit Natronlauge erhielt man schließlich das gewünschte Dihydroxybenzamid.

In Tabelle 1 sind einige der nach diesem Verfahren hergestellten Produkte aufgeführt:

Tabelle 1

| eingesetztes Amin | Dihydroxybenzamid gemäß Formel I | Schmelzpunkt (°C) |
|---|---|---|
| Anilin | N-Phenyl-2,4-dihydroxybenzamid · 0,5 $H_2O$ | 142 |
| 2-Methoxyanilin | N-(2'-Methoxyphenyl)-2,4-dihydroxybenzamid · 2 $H_2O$ | 60 - 65 |
| 3-Methoxyanilin | N-(3'-Methoxyphenyl)-2,4-dihydroxybenzamid · $H_2O$ | 115 - 120 |
| 4-Methoxyanilin | N-(4'-Methoxyphenyl)-2,4-dihydroxybenzamid | 209 - 210 |
| 4-Aminobenzoesäure | N-(4'-Carboxyphenyl)-2,4-dihydroxybenzamid · 0,5 $H_2O$ | > 250 |
| 2-Aminopyridin | N-(2'-Pyridyl)-2,4-dihydroxybenzamid · 1,5 $H_2O$ | 210 - 230 |
| 3-Aminopyridin | N-(3'-Pyridyl)-2,4-dihydroxybenzamid · 0,5 $H_2O$ | > 250 |
| 2,5-Dimethoxyanilin | N-(2',5'-Dimethoxyphenyl)-2,4-dihydroxybenzamid · 2 $H_2O$ | 135 - 136 |
| 3,5-Dimethoxyanilin | N-(3',5'-Dimethoxyphenyl)-2,4-dihydroxybenzamid · 2,5 $H_2O$ | 165 - 166 |
| 2-Methoxy-5-aminoanilin | N-(2'-Methoxy-5'amino-phenyl)-2,4-dihydroxybenzamid · $H_2O$ | 230 - 235(Z) |
| 4-N,N-Dimethylaminoanilin | N-(4'-(N,N,-Dimethylamino)phenyl)-2,4-dihydroxybenzamid · 3 $H_2O$ | 252 (Z) |
| 4-Aminophenol | N-(4'-Hydroxyphenyl)-2,4-dihydroxybenzamid · $H_2O$ | 228 - 230 (Z) |
| Methylamin | N-Methyl-2,4-dihydroxybenzamid | 188 - 190 |
| Benzylamin | N-Benzyl-2,4-dihydroxybenzamid · 0,5 $H_2O$ | 84 - 85 |

Fortsetzung Tabelle 1

| eingesetztes Amin | Dihydroxybenzamid | Schmelzpunkt (°C) |
|---|---|---|
| Anilin | N-Phenyl-3,5-dihydroxybenzamid | 112 - 114 |
| Anilin | N-Phenyl-2,5-dihydroxybenzamid · 0,5 $H_2O$ | 190 - 192 |
| Anilin | N-Phenyl-2,6-dihydroxybenzamid | 200 - 205 |
| Ammoniak | 2,4-Dihydroxybenzamid · 0,5 $H_2O$ | 220 (Z) |
| N-Methylanilin | N-Methyl-N-Phenyl-2,4-dihydroxybenzamid · $H_2O$ | 140 - 143 |
| N-Methyl-4-methoxyanilin | N-Methyl-N-(4'-methoxyphenyl)-2,4-dihydroxybenzamid | 148 - 150 |
| 3-Aminophenol | N-(3'-Hydroxyphenyl)-2,6-dihydroxybenzamid · 0,5 $H_2O$ | 230 - 236 (Z) |
| 4-Aminophenol | N-(4'-Hydroxyphenyl)-2,6-dihydroxybenzamid | 230 - 235 (Z) |

## II. Anwendungstechnische Prüfungen

Es wurden erfindungsgemäße Haarfärbemittel in Form einer Haarfärbe-Cremeemulsion der folgenden Zusammensetzung hergestellt:

| | |
|---|---|
| Fettalkohol $C_{12-14}$ | 10,0 g |
| Fettalkohol $C_{12-14}$ + 2 Ethylenoxid - sulfat, Na-Salz, 28%ig | 25,0 g |
| Wasser | 60,0 g |
| 2,4,5,6-Tetraaminopyrimidin | 7,5 mMol |
| Kupplerkomponente | 7,5 mMol |
| $Na_2SO_3$ (Inhibitor) | 1,0 g |
| konzentrierte Ammoniak-Lösung | bis pH = 9,5 |
| Wasser | ad 100 g |

Die Bestandteile wurden die Reihe nach miteinander vermischt. Nach Zugabe der Oxidationsfärbemittelvorprodukte und des Inhibitors wurde zunächst mit konzentrierter Ammoniak-Lösung der pH-Wert der Emulsion auf 9,5 eingestellt, dann wurde mit Wasser auf 100 g aufgefüllt.

Die oxidative Entwicklung der Färbung wurde mit 3%iger Wasserstoffperoxidlösung als Oxidationsmittel durchgeführt. Hierzu wurden 100 g der Emulsion mit 50 g Wasserstoffperoxidlösung (3%ig) versetzt und vermischt.

Die Färbecreme wurde auf ca. 5 cm lange Strähnen standardisierten, zu 90 % ergrauten, aber nicht besonders vorbehandelten Menschenhaars aufgetragen und dort 30 Minuten bei 27 °C belassen. Nach Beendigung des Färbeprozesses wurde das Haar gespült, mit einem üblichen Haarwaschmittel ausgewaschen und anschließend getrocknet. Die mit den genannten Oxidationsfärbemittelvorprodukten erhaltenen Farbnuancen der gefärbten Haarsträhne sind Tabelle 2 zu entnehmen.

Tabelle 2

| Kupplerkomponente | Farbnuance |
|---|---|
| N-Phenyl-2,4-dihydroxybenzamid | violettbraun |
| N-(2'-Methoxyphenyl)-2,4-dihydroxybenzamid | violettblau |
| N-(3'-Methoxyphenyl)-2,4-dihydroxybenzamid | dunkelrubin |
| N-(4'-Methoxyphenyl)-2,4-dihydroxybenzamid | blauviolett |
| N-(4'-Carboxyphenyl)-2,4-dihydroxybenzamid | rotbraun |
| N-(2'-Pyridyl)-2,4-dihydroxybenzamid | rotbraun |
| N-(3'-Pyridyl)-2,4-dihydroxybenzamid | rotbraun |
| N-(2',5'-Dimethoxyphenyl)-2,4-dihydroxybenzamid | blauviolett |
| N-(3',5'-Dimethoxyphenyl)-2,4-dihydroxybenzamid | blauviolett |
| N-(2'-Methoxy-5'amino-phenyl)-2,4-dihydroxybenzamid | schwarzviolett |
| N-(4'-(N,N,-Dimethylamino)phenyl)-2,4-dihydroxybenzamid | violett |
| N-(4'-Hydroxyphenyl)-2,4-dihydroxybenzamid | blauviolett |
| N-Methyl-2,4-dihydroxybenzamid | rot |
| N-Benzyl-2,4-dihydroxybenzamid | violettbraun |
| 2,4-Dihydroxybenzamid | orangebraun |
| N-Methyl-N-Phenyl-2,4-dihydroxybenzamid | grauviolett |

Fortsetzung Tabelle 2

| Kupplerkomponente | Farbnuance |
|---|---|
| N-Methyl-N-(4'-methoxyphenyl)-2,4-dihydroxybenzamid | graurot |
| N-Phenyl-3,5-dihydroxybenzamid | violettbraun |
| N-Phenyl-2,5-dihydroxybenzamid | violettbraun |
| N-Phenyl-2,6-dihydroxybenzamid | braun* |
| N-(3'-Hydroxyphenyl)-2,6-dihydroxybenzamid | braun |
| N-(4'-Hydroxyphenyl)-2,6-dihydroxybenzamid | braun |

* = mit p-Toluylendiamin als Entwicklerkomponente

## Ansprüche

1. Haarfärbemittel, enthaltend Oxidationsfarbstoffvorprodukte mit üblichen Entwicklerkomponenten in einem kosmetischen Träger, dadurch gekennzeichnet, daß sie als Kupplerkomponenten Dihydroxybenzamide der allgemeinen Formel (I)

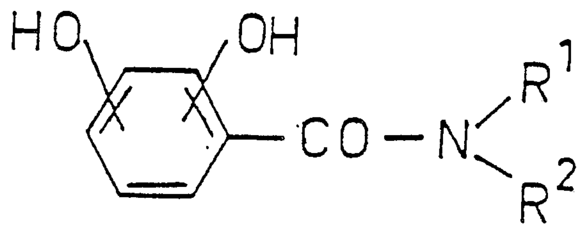

(I)

enthalten, in der $R^1$ und $R^2$ unabhängig voneinander Wasserstoff oder Alkylgruppe mit 1-4 Kohlenstoffatomen, Hydroxyalkyl- oder Aminoalkyl-Guppen, jeweils mit 2-4 Kohlenstoffatomen, Pyridyl-Gruppen oder Gruppen der Formel (II)

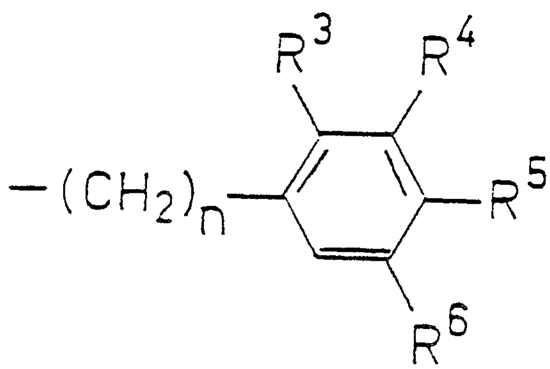

(II)

darstellen, wobei n = 0 - 4 ist und $R^3$-$R^6$ für Wasserstoff oder Hydroxylgruppen, Alkyl- oder Alkoxygruppen mit 1-4 Kohlenstoffatomen, Hydroxyalkyl- oder Aminoalkyl-Gruppen, jeweils mit 2-4 Kohlenstoffatomen, für $NR^7R^8$-Gruppen, mit $R^7$ und $R^8$ für Wasserstoff, oder Alkylgruppen mit 1-4 Kohlenstoffatomen, Hydroxyalkyl- oder Aminoalkylgruppen, jeweils mit 2-4 Kohlenstoffatomen, oder für die Carboxylgruppe stehen, wobei wenigstens zwei der Reste $R^3$-$R^6$ Wasserstoffatome sind und nicht mehr als ein Rest eine Carboxylgruppe ist.

2. Haarfärbemittel nach Anspruch 1, dadurch gekennzeichnet, daß sie als Kupplerkomponenten Dihydroxybenzamide, entsprechend der allgemeinen Formel (I), enthalten, bei denen die Hydroxylgruppen in den Positionen 2 und 4 stehen.

3. Haarfärbemittel nach Anspruch 1 oder 2, dadurch gekennzeichnet, daß sie als Kupplerkomponenten Dihydroxybenzamide der allgemeinen Formel (I) enthalten, in der $R^1$ Wasserstoff und $R^2$ Wasserstoff, eine Methylgruppe, eine Pyridyl-Gruppe oder eine Phenylgruppe der Formel (II) darstellen, wobei $R^3$-$R^6$ stehen für Wasserstoff, oder Methoxygruppen, Aminogruppen, N,N-Dimethylaminogruppen, oder die Carboxylgruppe unter der Bedingung, daß mindestens zwei dieser Gruppen $R^3$-$R^6$ Wasserstoffatome sind und das Molekül maximal eine Carboxylgruppe enthält.

4. Haarfärbemittel nach einem der Ansprüche 1 bis 3, dadurch gekennzeichnet, daß sie als Entwicklerkomponenten 2,4,5,6-Tetraaminopyrimidine der allgemeinen Formel (III)

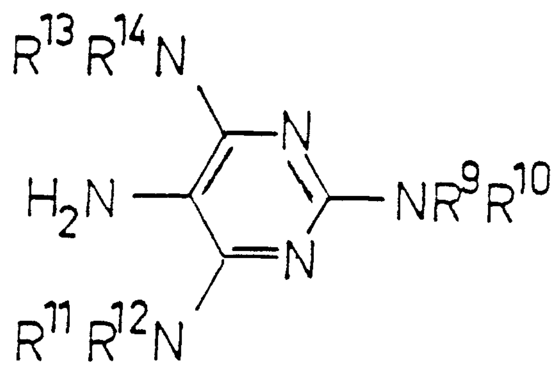

(III)

enthalten, in der $R^9$-$R^{14}$ Wasserstoff, einen Alkylrest mit 1-4 Kohlenstoffatomen, den Rest $-(CH_2)_m-X$, in dem m = 1-4 und X eine Hydroxylgruppe, ein Halogenatom, eine $-NR^{15}R^{16}$-Gruppe, wobei $R^{15}$ und $R^{16}$ Wasserstoff oder Alkylrest mit 1-4 Kohlenstoffatomen bedeuten können, darstellt, oder unter der Bedingung, daß $R^{11}$-$R^{14}$ Wasserstoffatome sind, $-NR^9R^{10}$ steht für eine Gruppe (IV)

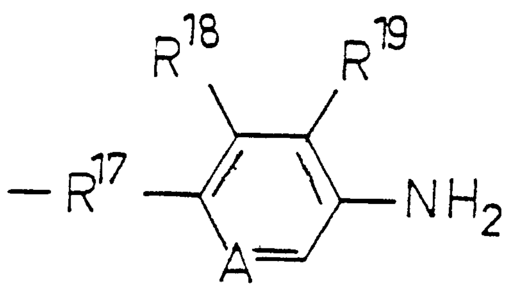

(IV)

in der $R^{17}$ eine Gruppe $-NH-(CH_2)_p-NH-$, worin p = 2 - 4 ist, eine Gruppe $-NH-CH_2-CH(OH)-CH_2-NH-$ oder eine Gruppe

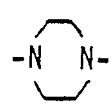

ist, $R^{18}$ und $R^{19}$ unabhängig voneinander Wasserstoff, Chlor oder eine Gruppe $-OR^{20}$ sind, worin $R^{20}$ eine Alkylgruppe mit 1 bis 4 C-Atomen ist, und A eine CH-Gruppe oder ein Stickstoffatom ist,
oder $R^9$ und $R^{10}$ und/oder $R^{11}$ und $R^{12}$ und/oder $R^{13}$ und $R^{14}$ mit dem jeweiligen Stickstoffatom einen heterocyclischen, 5- oder 6-gliedrigen Ring mit einem oder zwei Stickstoffatomen oder einem Stickstoff- und einem Sauerstoffatom bilden können, und/oder deren Salze mit anorganischen oder organischen Säuren.

5. Haarfärbemittel nach einem der Ansprüche 1 bis 4, dadurch gekennzeichnet, daß sie als Entwicklerkomponente 2,4,5,6-Tetraaminopyrimidin enthalten.

6. Haarfärbemittel nach einem der Ansprüche 1 bis 5, dadurch gekennzeichnet, daß sie 0,2-5 Gew.-%, insbesondere 1-3 Gew.-%, an Oxidationsfarbstoffvorprodukten enthalten.

Europäisches Patentamt

# EUROPÄISCHER RECHERCHENBERICHT

Nummer der Anmeldung

EP 89 11 1560

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X,Y | DE-A-2 717 041 (HENKEL)<br>* Insgesamt *<br>--- | 1-6 | A 61 K 7/13 |
| Y | EP-A-0 142 241 (KUREHA KAGAKU KOGYO K.K.)<br>* Ansprüche *<br>--- | 1-6 | |
| Y | EP-A-0 250 723 (FUJI PHOTO FILM CO.)<br>* Anspruch 1 *<br>--- | 1-6 | |
| A | EP-A-0 026 473 (WELLA)<br>* Insgesamt *<br>--- | 1-6 | |
| A,D | FR-A-2 353 600 (AMERICAN COLOR & CHEMICAL CORP.)<br>* Anspruch 1 *<br>----- | 1 | |

RECHERCHIERTE SACHGEBIETE (Int. Cl.5)

A 61 K
C 07 C

Der vorliegende Recherchenbericht wurde für alle Patentansprüche erstellt

| Recherchenort | Abschlußdatum der Recherche | Prüfer |
|---|---|---|
| DEN HAAG | 06-10-1989 | FISCHER J.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)